# EUROPEAN PATENT APPLICATION

(11) **EP 0 844 474 A2**
(43) Date of publication of application: **27.05.1998**
(21) Application number: 97309262.0
(22) Date of filing: 18.11.1997
(51) Int. Cl.: G01N 11/14, G01N 33/44

(54) **Method of measuring rheological properties of a material and device therefor**

(30) Priority: 21.11.1996 GB 9624264
(71) Applicant: The Malaysian Rubber Producers' Research Association, Brickendonbury, Hertfordshire, SG13 8NL (GB)
(72) Inventor: Fulton, Willaim S., The Malaysian Rubber Producers, Brickendonbury, Hertford, SG13 8NL (GB); Stephens, Ian J., The Malaysian Rubber Producers, Brickendonbury, Hertford, SG13 8NL (GB)
(74) Representative: Perkins, Sarah

(57) **Abstract**

The method of measuring rheological properties of a material is particularly suited to measuring the properties of natural rubber to assist in predicting the behaviour of the material during processing. The method may also be used as a quality control test. With the method resistance to the rotation of a rotor head 12 in chamber 20 containing the natural rubber is measured before and after the natural rubber has been subjected to a shear rate of around 100 sec ⁻¹ for a predetermined period of time. In this way breakdown indices which may be termed a modified Mooney difference breakdown index and a modified Mooney ratio breakdown index are derived from the measurements. The results have been found to be particularly reliable in providing a strong correlation with the effects produced in an industrial mixing process.

## Description

The present invention relates to a method of measuring rheological properties of a material and a device therefor. In particular, but not exclusively, the present invention relates to the measurement of the rheological properties of natural rubber whereby the behaviour of a particular batch of natural rubber during processing may be approximately predicted. The present invention also relates to a device for carrying out such a method which enables measurement of rheological properties to be carried out quickly and easily, for example as a quality control test, either by rubber producers or by manufacturers who are consumers of natural rubber.

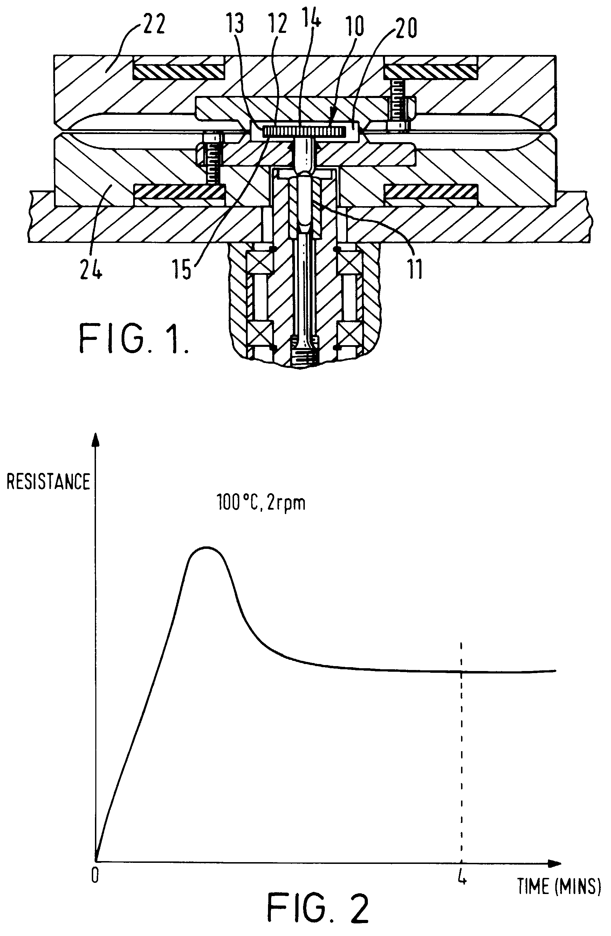

Mooney viscometers have been used for some time to measure the viscosity of a sample of rubber in the production and processing of natural rubber. Figure 1 shows part of a conventional Mooney viscometer which comprises a rotor 10 rotatably mounted within a sealable chamber 20. The rotor 10 has a disc-shaped head 12 with a knurled edge 13 and roughened upper 14 and lower 15 surfaces which provide surfaces having good frictional properties for contact with a sample of rubber when placed within the chamber 20. The rotor head 12 is provided on a shaft 11 of non-circular (usually of square or rectangular) cross-section which is mounted for rotation within the chamber 20.

Rotation of the rotor is driven, typically through a worm and worm wheel gear arrangement (not shown in Figure 1). The worm is mounted in journal bearings which allow limited movement along the direction of its axis. Any resistance to the rotation of the worm wheel generates a torque at the point of intersection of the worm and the worm wheel, which in turn generates a thrust axially directed along the worm. This thrust is borne by a strong spring-mounted pad the displacement of which is proportional to the torque generated by the resistance to rotation of the worm wheel. The displacement of the pad is measured by any suitable means such as a transducer.

In order to measure the viscosity of a sample of rubber, a thin slice of the rubber sample, having the same shape as the rotor head 12, is located adjacent the underside 15 of the rotor head and another, similarly shaped slice of the sample is placed on the upper side 14 of the rotor head. The chamber 20 within which the rotor head 12 is located is formed by upper 22 and lower 24 platens. The upper platen 22 is raised to allow the slices of rubber to be placed about the rotor head 12. Thereafter, the upper platen 22 is lowered, sealing the chamber 20 and applying pressure to the slices of rubber within the chamber such that the rubber flows to completely fill the space between the rotor head and the internal walls of the chamber.

The conventional Mooney viscosity test then comprises heating the rubber sample within the chamber 20 to 100°C and rotating the rotor 10 at a rate of about 2 revolutions per minute (rpm), for approximately four minutes. The viscosity of the rubber sample causes resistance to the rotation of the rotor head 12 within the chamber 20 which in turn causes a torque to be generated at the intersection between the worm and the worm wheel. The size of the torque is used as a measure of the viscosity of the sample.

Figure 2 is a diagrammatic graph showing how resistance to the rotation of the rotor head (as measured by any suitable measuring means) varies throughout a typical Mooney viscosity test. It can be seen that by four minutes of rotation of the rotor at 2 rpm, the resistance to the rotor head 12 is substantially constant. Hence, after about 4 minutes a reading is taken of the torque (in arbitrary units) which provides a measure of the viscosity of the rubber sample.

It will, of course, be appreciated that the essence of the Mooney viscometer test is that it provides a measure of viscosity which is used to compare samples. Traditionally, certain types of natural rubber have been graded by performing this test.

The Mooney viscosity test does not though give an indication of how rubber will behave during processing. That is to say, different batches of rubber may generate identical results in a Mooney viscosity test, but may behave differently during processing. Quite clearly this can adversely affect the consistency of final products manufactured from rubber taken from different batches.

A test known as the Brabender Plasticorder Test has been suggested in which the initial viscosity of a rubber sample is measured using the Mooney viscosity test. Thereafter, the remaining portion of the sample (usually about 60g) is masticated in a mixer at a starting temperature of 100°C for 4 minutes. After mastication, the resulting viscosity of the masticated sample is measured, again employing the Mooney viscosity test. A breakdown index is calculated from the difference in measured viscosity before and after mastication of the rubber and from the amount of energy expended in masticating the rubber. The mixer used in this test simulates a typical stage in the processing of natural rubber.

However, the Brabender Plasticorder Test suffers from a number of disadvantages which have prevented it from being adopted by the rubber industry at large as a standard quality control test to complement the Mooney viscosity test. Firstly, the equipment necessary to perform this test (especially the mixer which simulates a typical large scale mixer used in rubber processing) has proved prohibitively expensive for rubber producers and processors alike. Secondly, the test is time consuming because after mastication the rubber sample has to be allowed to cool and then removed from the mixer before a second Mooney viscosity test may be carried out. The necessity to take samples of samples and to have to transfer the samples between separate devices also introduces practical complications which are not suited to quality control tests in general.

The present invention seeks to provide a method of measuring rheological properties of a material, and a device therefor, which overcomes at least some of the disadvantages associated with the prior art discussed above.

According to a first aspect of the present invention, there is provided a method of measuring rheological properties of a material including the steps of:
locating a sample of material in contact with a rotor head within a chamber;
rotating the rotor head at a first substantially constant rate of rotation for a first predetermined period of time;
measuring the resistance to rotation of the rotor head;
increasing the rate of rotation of the rotor head, to a second rate of rotation, for a second predetermined period of time;
rotating the rotor head at a third substantially constant rate of rotation, which is less than said second rate of rotation, for a third predetermined period of time; and
measuring the resistance to rotation of the rotor head.

Preferably the first and third rates of rotation are substantially equal and the second rate of rotation is about two orders of magnitude greater than the first and third rates of rotation. The currently preferred second rate of rotation is such as to correspond to a shear rate of approximately 100 sec⁻¹.

Prior to the step of rotating the rotor head at said first rate of rotation, the method may further include the step of heating the sample of material to between 80°C and 200°C, preferably 100°C (however in the case of synthetic rubber there may be merit in heating the sample to a temperature slightly below 80°C). After the step of rotating the rotor head at said second rate of rotation, the method may further include the step of cooling the sample to approximately the same temperature as the temperature of the sample immediately before rotation of the rotor head at said first rate of rotation, before commencing the next step. Preferably, the sample is left to cool whilst continuing rotation of the rotor head at said third rate of rotation.

Preferably, the method further includes the step of calculating the difference between the two measurements taken during the test. Alternatively, or in addition, the method may further include the step of calculating a ratio of the two measurements taken during the test.

According to a second aspect of the present invention, there is provided a device for measuring rheological properties of a material including a rotor having a rotor head; a chamber within which the rotor head may be mounted; a first driving means connectable to the rotor for rotating said rotor at a first rate of rotation; a second driving means connectable to the rotor for rotating said rotor at a second rate of rotation, which is greater than said first rate of rotation; a third driving means connectable to the rotor for rotating said rotor at a third rate of rotation, which is less than said second rate of rotation; drive control means for ensuring at any one time that only one of the first, second and third driving means is rotating said rotor, and measurement means for measuring resistance to the rotation of the rotor.

Preferably, the first and third driving means consist of a single motor whereby the first and third rates of rotation of the rotor may be the same. Also, the first, second and third driving means may consist of a single variable speed motor, or a single one speed motor connected to the rotor via gearing mechanisms which give rise to the necessary different rates of rotation. Clutch means may be provided to ensure that only one gearing mechanism connects the motor to the rotor at any one time. It is preferable that a single variable speed motor is not used to achieve both the first/third as well as the second rate of rotation because there is preferably a difference of at least two orders of magnitude between these rates. A single variable speed motor able to provide these different speeds would be expensive and there would be considerable inefficiency owing to wasted capacity between the extremes.

In a preferred embodiment the first and third driving means comprise a motor which is separate from the second driving means comprising a second motor, the first motor being connected to the rotor through worm gearing with the worm being axially biased such that the axial displacement of the worm is measured by the measurement means. The measurement means may therefore comprise a displacement transducer.

The drive control means may include clutch means for coupling or disconnecting the first and second motors sequentially. In this preferred embodiment, the clutch means preferably comprises a first sprag clutch connecting the first motor through the worm gearing to the rotor and a second sprag clutch connecting the second motor to the rotor with both sprag clutches being orientated such that whichever motor is driving most quickly in a selected direction is coupled to the rotor with the other sprag clutch isolating the rotor from the other motor such that only a minimal amount of drag from the other motor is transmitted to the rotor.

In the preferred embodiment the first sprag clutch is lubricated by a lubricating fluid bath which is located beneath the worm and the radially inner edge of the first sprag clutch. The worm may be arranged so that the lowermost part of the worm dips into the lubricating fluid bath. Additionally, lubricating fluid collected by the lowermost part of the worm may be caused to fall on the sprag clutch as the worm rotates thereby lubricating the sprag clutch.

The device preferably includes heating means for heating the chamber. The heating means is preferably located in either an upper or lower platen, or in both, and may take the form of a plurality of electrical heating elements.

According to a third aspect of the present invention, there is provided a method of adapting a conventional Mooney viscometer having a rotor, a worm wheel attached to the rotor and a worm for driving the worm wheel, said method comprising the steps of replacing the worm wheel on the rotor with a first sprag clutch connected to a worm wheel, coupling a second sprag clutch to said rotor and connecting a second driving means to said second sprag clutch.

In order that the present invention may be better understood, embodiments thereof will now be described, by way of example only, with reference to the accompanying drawings in which:-
Figure 1 is a diagrammatical, cross-sectional view of a part of a conventional Mooney viscometer;
Figure 2 is a diagrammatical graph showing the resistance to rotation of a rotor head with respect to time for a typical, conventional Mooney viscosity test;
Figure 3 is a diagrammatical, cross-sectional view of a device for measuring rheological properties of rubber according to the present invention;
Figure 4 is a diagrammatical graph, similar to that shown in Figure 2, showing the resistance to rotation of a rotor head with respect to time for a method according to the present invention;
Figure 5 is a diagrammatical graph comparing a breakdown index arising from results obtained employing the method according to the present invention against a similar breakdown index determined by measuring the viscosity of different types of rubber before and after a conventional rubber processing operation;

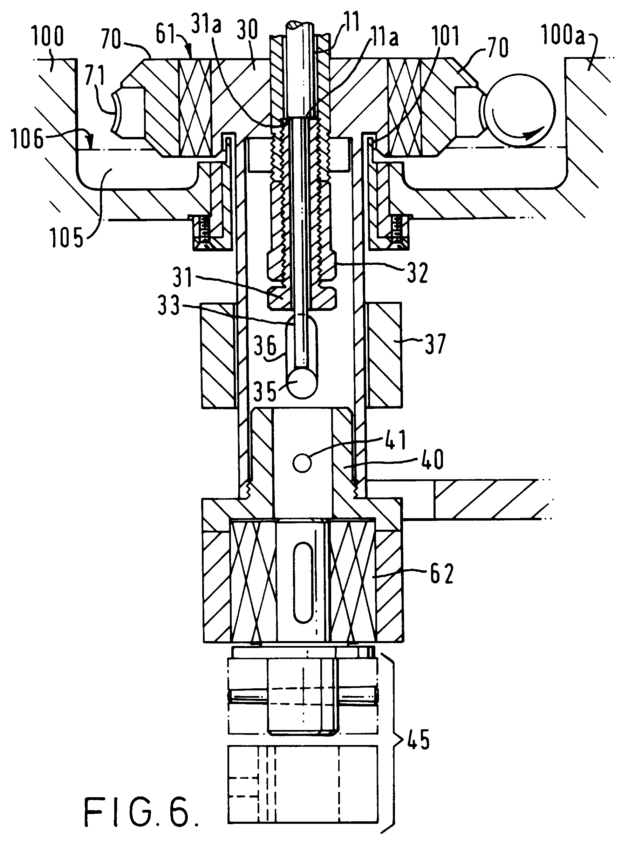

Figure 6 is a diagrammatical, exploded view of a part of the device shown in Figure 3; and
Figure 7 is a diagrammatical plan view of a worm and worm wheel gear arrangement, suitable for use with the present invention.

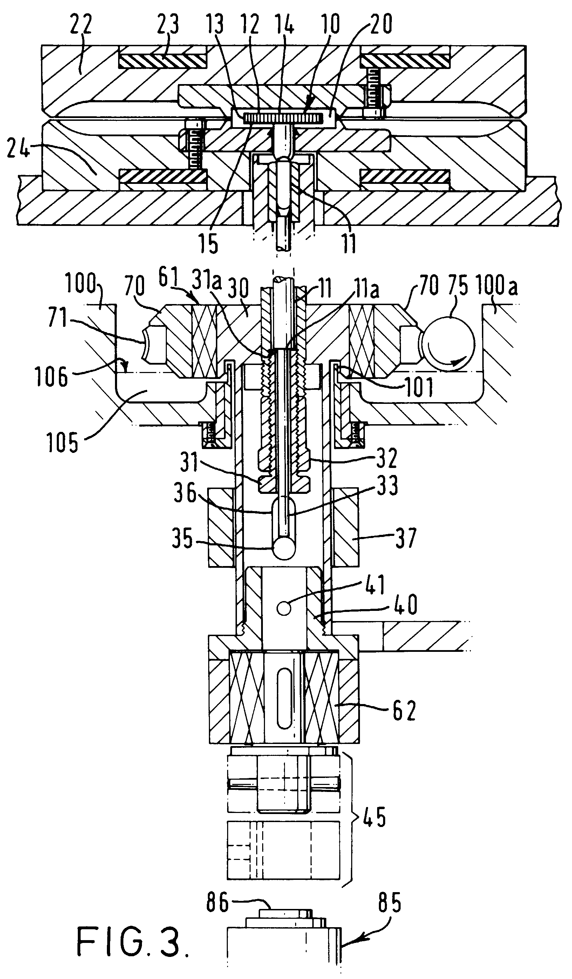

Referring to Figure 3, the device for measuring rheological properties of an elastomeric sample has a rotor 10 rotatably mounted within a sealable chamber 20. The sealable chamber 20 is formed within upper 22 and lower 24 platens. The rotor 10 has a rotor head 12 which will typically be disc-shaped as is shown in Figure 3, although it may have an alternative shape specially adapted for different purposes. Typically, there may be a set of slightly different rotors 10 having different characteristics all of which may be used in the device for different purposes. The rotor 10 shown has a knurled edge 13 and roughened upper 14 and lower 15 surfaces. In this way, the surfaces 13,14,15 which will be in contact with a sample of rubber when placed in the chamber 20 have good frictional properties to prevent or reduce slippage of the rubber with respect to the rotor head 12. The rotor head 12 is mounted on a shaft 11 of non-circular (for example, of square or rectangular) cross-section which is mounted for rotation with, or is integral with, the rotor head 12.

The chamber 20 is formed by the upper 22 and lower 24 platen, the upper platen of which may be raised to gain access to the chamber for preparing, for example, a sample of rubber to be tested, or for removing a rotor and attached sample after a test has finished. Heating means, in the form of electrical heating elements 23 are provided in the platens 22,24, to enable the contents of the chamber 20 to be raised to a desired temperature.

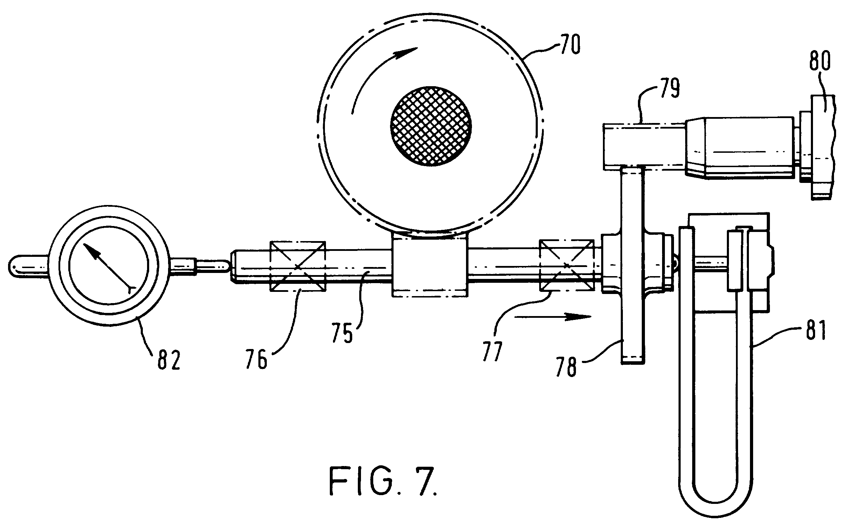

Referring now to Figure 7 as well as to Figure 3, it can be seen that the rotor 10 may be driven by a worm 75 and worm wheel 70 arrangement, the worm 75 being driven in turn by a first driving means in the form of a motor 80 for example. Alternatively, the rotor 10 may be driven by a second driving means or motor 85 which is adapted to drive the rotor 10 at a much greater rate of rotation than the first motor 8 in a manner described in greater detail below. As may be seen from Figure 7, the worm 75 is mounted in journal bearings 76,77 which allow limited movement of the worm along the direction of its axis with minimal frictional resistance. A spur gear wheel 78 and pinion 79 are used to drive the worm wheel 75 from the first motor 85 so as to enable the majority of any thrust generated along the axis of the worm 75 to be borne by a U-spring 81. Any axial displacement of the worm 75 is registered by the dial gauge 82 or other suitable means such as a transducer and associated electronics.

The rotor shaft 11 is fitted to be able to slide axially within an axial bore through a sleeve drive shaft 30. The base 11a of the rotor shaft 11 abuts against a rotor shaft release device in the form of an axially upwardly facing abutment surface 31a formed on a rotor adjustment sleeve bolt 31, which, together with a locking nut 32 is mounted by a helical screw-thread fit within the sleeve drive shaft 30. Axially slidably located within the sleeve bolt 31 is an ejector pin 33 which rests on a crossbar pin 35. The crossbar pin 35 extends radially outwardly through opposing longitudinal grooves 36, formed in the sleeve drive shaft 30, where it engages with a collar 37 which is mounted around the sleeve drive shaft 30 and is axially slidable therewith.

A drive shaft extension 40 is mounted onto the sleeve drive shaft 30 by means of co-operating screw-threads formed on the inner and outer surfaces of the sleeve drive shaft 30 and the drive shaft extension 40 respectively. When the drive shaft extension 40 has been tightly screwed into place, a tapered pin 41 is driven between the sleeve drive shaft 30 and the drive shaft extension 40 to hold them securely in place.

The sleeve drive shaft 30 is connected to the worm gear arrangement via clutch means in the form of a first sprag clutch assembly 61. The worm gear consists of a worm wheel 70 which has a thread pattern formed on its circumference 71 which co-operates with a thread pattern provided on a worm 75 mounted horizontally, tangentially to the worm wheel 70. Rotation of the worm 75 drives the worm wheel 70 about its respective axis. The worm 75 is driven by the first motor 80. A small reservoir or bath 105 having high temperature lubricating oil 106 located therein is provided underneath the worm wheel 70 and first sprag clutch assembly 61. The reservoir 105 is defined by a casing 100 and a small circumferential flange 101 which extends axially upwardly, radially inwardly of the first sprag clutch assembly 61.

The drive shaft extension 40 is connected to a second motor shaft 86 which is driven by the second motor 85, via second clutch means which is preferably a second sprag clutch assembly 62 and a coupling assembly 45. The second motor is selected to drive the rotor shaft 11 at a rate of rotation much greater than the rate of rotation of the rotor shaft 1 1 when driven by the first motor.

The first and second sprag clutch assemblies 61 and 62 are conventional and include inner and outer raceways with a combination of balls (or rollers) and pivotal wedges located in respective axially separated circumferential tracks. These clutches, as is well known in the art, have the function of bearings for one direction of relative rotation between the inner and outer raceways and provide a direct coupling between the raceways when caused to rotate in the opposite relative direction. For example, the first sprag clutch 61 may be arranged so that with the inner raceway rotating anticlockwise at a speed greater than the anticlockwise rotation of the outer raceway (i.e. the relative rotation of the outer raceway is clockwise), the clutch acts as a substantially frictionless bearing whereas with the inner raceway rotating anticlockwise at a speed less than the anticlockwise rotation of the outer raceway (i.e. the relative rotation of the inner raceway is clockwise), the clutch provides a direct coupling between the inner and outer raceways. The second sprag clutch 62, on the other hand in the arrangement shown in Figure 3, will be arranged in the opposite orientation so that a direct coupling is provided when the relative rotation of the outer raceway is clockwise and the raceways act as frictionless bearings when the relative rotation of the inner raceway is clockwise.

Referring to Figures 3 and 7, when the first motor 80 is energised and drives the outer raceway of the first clutch 61 through the worm gear arrangement 70,75 anticlockwise, initially the speed of rotation of the inner raceway is less than that of the outer raceway and so the raceways form a direct coupling which causes the inner raceway and so the rotor shaft 11 to rotate anticlockwise at the same rate of rotation. If the second motor 85 is then energised to cause the rotor shaft 11, via the drive shaft extension 40, to rotate anticlockwise at a speed greater than the speed of the rotor 10 when driven by the first motor 80, the inner and outer raceways of the first clutch 61 disengage and now function as substantially frictionless bearings. In this way when the second motor engages the rotor shaft 11 via the second clutch 62, the first motor 80 is automatically disengaged by the action of the first sprag clutch 61 so that the sleeve drive shaft 30 experiences very little drag from the worm gear arrangement 70,75 when driven by the second motor 85. Similarly, the second sprag clutch 62 ensures that when the second motor 85 is not energised it is isolated from rotation of the drive shaft extension 40 and thereby ensures the rotor shaft 11 experiences almost no drag when driven by the first motor 80.

The overall effect of this arrangement, it will be seen, is that the rotor shaft 11 is driven at a rate determined by whichever of the worm wheel 70 or the second motor shaft 86 is driven more quickly, the relevant clutch in each case effectively disengaging the slower element from the drive shaft by behaving essentially as a low friction bearing.

The first sprag clutch 61 is located close to the chamber 20 where a rubber sample to be tested is located. During operation of the device, the chamber 20 is heated to within the range 80°C to 200°C but preferably to approximately 100°C and the temperature within the chamber 20 may rise even higher during high speed rotation of the rotor 10. Furthermore, when the drive shaft 30 is driven at high speeds by the second motor, the inner and outer raceways of the first sprag clutch 61 are rotating at a high relative rate with respect to one another. Both of these factors combine to render elastomeric seals unsuitable for sealing the first sprag clutch 61 because of the high operating temperature of the clutch 61. This generates a problem with regards to the lubrication of the first sprag clutch 61.

To overcome this problem, the lubricating oil bath 105 is provided beneath the first sprag clutch 61 to keep the clutch well lubricated. The worm 75 has a sufficiently large diameter to "dip" into the oil bath 105 thus providing constant lubrication between the worm 75 and worm wheel 70. Furthermore, some oil will be spun off the worm 75 as it rotates; so the direction of rotation of the worm 75 is chosen so as to ensure that some of the oil spun from the worm 75 lands above the sprag clutch 61 and seeps therethrough. Since the inner raceway of the sprag clutch 61 rotates relative to the outer raceway and the worm 75 when the rotor shaft 11 is operating at high speed, and thus is in most need of lubrication, the sprag clutch 61 is kept well lubricated at all times. The flange 101 on the casing 100 acts as a splash break to prevent oil from the bath 105 leaking out down the sleeve drive shaft 30.

It is possible to modify an existing Mooney viscometer in order to make a device as shown in Figure 3. To do this the existing worm wheel 70 is removed. The internal diameter of the worm wheel 70 is then increased to accommodate suitable clutch means, preferably in the form of a sprag clutch 61 or alternatively, the worm wheel is replaced with a new worm wheel having the necessary internal diameter. In order to lubricate the sprag clutch 61 the housing 100 is widened 100a to accommodate the sprag clutch and the worm wheel and small modifications are made to the flange 101 to allow the level of an oil bath 106 underneath the worm wheel 70 to be increased. The flange 101 is thinned so as to reduce the capillary effect between the flange 101 and the drive shaft 30. This in turn reduces leakage down the drive shaft 30.

Additionally, the drive shaft 30 is extended downwards, or replaced with another having a sufficiently long downward extrusion to enable a second motor to be coupled to the shaft. A pair of opposite longitudinal slits 36 are formed in the drive which enable a crossbar pin 35 to rest underneath the ejector pin 33. As explained above, this is then attached to a sleeve 36 which rotates with the drive shaft 30 but is axially slidable therewith such that raising of the sleeve 36 will force the ejector pin 33 to eject the rotor 10. A drive shaft extension 40 may be used for coupling the second motor 85 to the drive shaft via clutch means preferably in the form of a second sprag clutch.

In order to calculate a breakdown index using this device, which the inventors have termed a modified Mooney viscometer, a process or test has been developed which the inventors have termed a modified Mooney breakdown test. According to this test two slices of a rubber sample to be measured are mounted on the rotor head 12 as is done with a conventional Mooney viscosity test. The rotor 10 is then fitted into the device and the upper platen 22 is lowered until the chamber 20 is sealed and the rubber sample has flowed under the pressure generated to substantially fill the chamber 20. The platens 22, 24 are then heated until the rubber sample is at approximately 100°C and the rotor is rotated at a rate of about 2 rpm, which corresponds to a shear rate of the rubber sample of about 1 sec⁻¹. The first motor is used to drive the rotor 10 at this low speed, thus enabling the resistance to the rotation of the rotor to be measured in the conventional way by measuring the axial displacement of the worm. After about 4 minutes of rotating the rotor at this low speed, a resistance measurement V_{R} is recorded.

Next the second motor is energised. This drives the rotor at a much faster rate of rotation which causes the rubber sample to breakdown because of the much increased rate of shearing. It is believed that the high shearing rate causes both mechanical and oxidative breakdown of the rubber. The rotor 10 at this stage is preferably driven at a rate of about 120 rpm which corresponds to a shear rate in the rubber sample of approximately 100 sec⁻¹ or an approximate increase with respect to a conventional Mooney viscosity test by two orders of magnitude. During this time, the first motor is kept running so that the first sprag clutch 61 is kept well lubricated by the 'splash over' effect described above. After about 1 minute the second motor is switched off or disconnected and the rotor 10 slows down to rotate at the rate dictated by the first motor. At this point the rubber sample will be significantly hotter than the 100°C at which the first viscosity reading was taken. The rubber sample is therefore allowed to cool back down to around 100°C. During the cooling period the first motor may be turned off or may continue driving the rotor. That is, the rotor 10 may or may not be rotating during the cooling period. After about 5 minutes there is sufficient cooling of the rubber sample, back to approximately 100°C, that a third step of rotating the rotor at a much lower rate via the worm and worm wheel arrangement and measuring the viscosity of the rubber in a similar manner as was done in the first step may now be performed. The cooling period may be usefully employed to apply the shear for which the usual torque measurements are subsequently taken. Of course, the cooling period could be reduced by taking active cooling steps such as blowing cool air about the chamber 20.

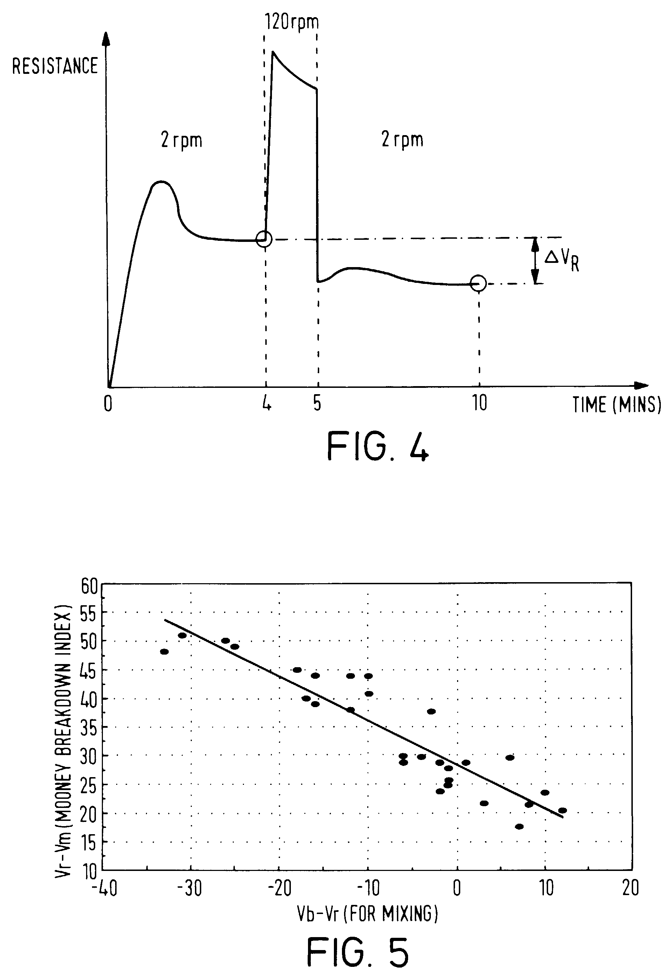

During the third principal step, the rotor is preferably driven at the same rate of rotation as it was driven in the first step such that a better comparative result will be achieved. However, the method will not be greatly affected if a different rate of rotation is used since the test is essentially a relative one and all that is important is that the entire test is applied consistently between batches of rubber to be tested. However, it is preferable for the rotation in the third step to be maintained approximately constant until a steady state of resistance is generated (see Figure 4) at which point a new measurement of viscosity V_{M} may be recorded.

From this procedure, simple breakdown indices can be derived from either the difference in the two viscosity measurements (i.e. ΔV_{R} = V_{R} - V_{M}) which may be termed a modified Mooney difference breakdown index or from the relative difference or ratio of the measurements (i.e. V_{R}/V_{M}) which may be termed a modified Mooney ratio breakdown index.

A number of different raw rubbers (latex and field grade) have been tested at various rotor speeds up to a maximum of 200 rpm, for various lengths of time, to determine which speed and duration gave the most discriminatory results, i.e. maximum relative breakdown. The greatest difference in results was found to occur at 120 rpm for one minute. However, speeds between 100-200 rpm for at least 0.5 minute may be employed to ensure satisfactory breakdown of the sample.

Of course, for the test to be of any real commercial significance, it is important that the indices derived in this way should relate to the behaviour of the same rubber when processed commercially. The chemical and structural alterations which occur to rubber when it is processed are not fully understood and there is thus no *a priori* or theoretical reasoning which would suggest that the breakdown caused to the rubber sample by subjecting it to a high shear rate, would bear any relation to the breakdown which rubber experiences when it is mixed in an internal mixer during a commercial processing stage. In particular, although it is probable that a rubber sample will be subjected to relatively high shearing in an industrial mixing process as a result of the mixing blades within the internal mixer, many other effects will also occur as a result of the mixing action of the mixing blades which is not in any way simulated in the process or test with which the present invention is concerned. However, it is surprisingly found that the breakdown which is caused by the second period of rotation as described above, does in fact provide a strong correlation with the effects produced in an industrial mixing process.

Figure 5 is a graph which compares results from the modified Mooney difference breakdown index and a breakdown index derived from the difference in viscosity measured after, and before, an industrial mixing process (V_{B} - V_{R}) for a number of different raw rubbers. In order to obtain the results for the latter index, the raw rubbers were mixed in a BR Banbury mixer to a formulation typical of a breaker compound (55 phr N330). From Figure 5 it can be seen that there is a correlation between raw rubber breakdown as measured by the modified Mooney breakdown test and the viscosity change on mixing (V_{B} - V_{R}) and confirms the premise that increased raw rubber breakdown will lead to easier compound mixing. More importantly, such results show how a modified Mooney viscometer can be used to predict the processability of natural rubber and thus bring the potential for an additional and more meaningful specification to standard raw rubber specifications such as embodied in the Standard Malaysian Rubber Scheme.

Although a preferred embodiment of the present invention has been described above, it will be appreciated by those skilled in the art that modifications may be made to the preferred embodiment. In particular, the shape of the rotor head may be altered, the manner in which the rotor is freed from the sleeve drive shaft and the arrangement for securing the sleeve drive shaft and the drive shaft extension may also be altered in ways immediately obvious to a person skilled in the art. Also, although two separate motors driving the rotor through sprag clutches are described herein it will be appreciated that alternative arrangements may be made whilst still enabling the rotor to be driven selectively at two, or three, very different speeds. The modified Mooney viscometer may be made by adapting or retrofitting an existing conventional Mooney viscometer or alternatively, dedicated machines may be manufactured. Also, the modified Mooney breakdown test described herein may be adjusted so as to fit in more easily with existing quality control tests for example by altering the lengths of time for which the various rotating stages are carried out, by using non-constant rates of rotation or by altering the temperatures at which they are carried out.

## Claims

1. A method of measuring rheological properties of a material including the steps of:
locating a sample of material in contact with a rotor head within a chamber;
rotating the rotor head at a first substantially constant rate of rotation for a first predetermined period of time;
measuring the resistance to rotation of the rotor head;
increasing the rate of rotation of the rotor head, to a second rate of rotation, for a second predetermined period of time;
rotating the rotor head at a third substantially constant rate of rotation, which is less than said second rate of rotation, for a third predetermined period of time; and
measuring the resistance to rotation of the rotor head.

2. A method as claimed in claim 1, wherein the first and third rates of rotation are substantially equal.

3. A method as claimed in either of claims 1 or 2, wherein the second rate of rotation is substantially two orders of magnitude greater than the first rate of rotation.

4. A method as claimed in claim 3, wherein the second rate of rotation corresponds to a shear rate of 100 sec⁻¹.

5. A method as claimed in claim 1, wherein prior to the step of rotating the rotor head at said first rate of rotation, the method includes the further step of heating the sample.

6. A method as claimed in claim 5, wherein the sample is heated between 80°C and 200°C.

7. A method as claimed in claim 6, wherein the sample is heated to around 100°C.

8. A method as claimed in claim 5, wherein the sample is heated to less than 80°C.

9. A method as claimed in claim 1, wherein after the step of rotating the rotor head at said second rate of rotation, the method includes the further step of cooling the sample to substantially the same temperature as the temperature of the sample immediately before rotation of the rotor head at said first rate of rotation.

10. A method as claimed in claim 9, wherein said sample is cooled whilst rotating the rotor head at said third rate of rotation.

11. A method as claimed in claim 1, further including the step of calculating the difference between the two measurements taken during the method.

12. A method as claimed in claim 1, further including the step of calculating a ratio of the two measurements taken during the method.

13. A device for measuring rheological properties of a material, the device including a rotor with a rotor head; a chamber within which the rotor head is mounted; a first driving means connectable to the rotor for rotating said rotor at a first rate of rotation; a second driving means connectable to the rotor for rotating said rotor at a second rate of rotation, which is greater than said first rate of rotation; a third driving means connectable to the rotor for rotating said rotor at a third rate of rotation, which is less than said second rate of rotation; drive control means for ensuring at any one time that only one of the first, second and third driving means is rotating said rotor, and measurement means for measuring resistance to the rotation of the rotor.

14. A device as claimed in claim 13, wherein the first and third driving means consist of a single motor whereby the first and third rates of rotation are substantially the same.

15. A device as claimed in either of claim 13 or 14, wherein the first, second and third driving means consist of a single variable speed motor.

16. A device as claimed in either of claims 13 or 14, wherein the first, second and third driving means consist of a single one speed motor connected to the rotor via selectable gearing.

17. A device as claimed in claim 14, wherein the first and third driving means comprise a first motor, separate from the second driving means that comprises a second motor.

18. A device as claimed in claim 17, wherein the first motor is connected to the rotor through a worm gear and in which the worm is axially biased such that axial displacement of the worm is measured by the measurement means.

19. A device as claimed in claim 18, wherein the measurement means is a displacement transducer.

20. A device as claimed in any one of claims 17 to 19, wherein clutch means are provided for coupling and disconnecting the first and second motors sequentially.

21. A device as claimed in claim 20, wherein the clutch means comprises first and second sprag clutches orientated so that whichever motor is driving the most quickly in a given direction is coupled to the rotor whilst the other of the two motors is isolated from the rotor.

22. A device as claimed in claims 18 and 21, wherein a lubricating fluid bath is provided for lubrication of the first sprag clutch and the worm gear.

23. A device as claimed in any one of claims 13 to 22, wherein a heating device is provided for heating the chamber.

24. A method of adapting a conventional Mooney viscometer having a rotor, a worm wheel attached to the rotor and a worm for driving the worm wheel, said method comprising the steps of replacing the worm wheel on the rotor with a first sprag clutch connected to a worm wheel, coupling a second sprag clutch to said rotor and connecting a second driving means to said second sprag clutch.
